(19) Europäisches Patentamt
European Patent Office
Office européen des brevets

(11) Publication number: 0 092 194 B1

# (12) EUROPEAN PATENT SPECIFICATION

(45) Date of publication of patent specification: 08.07.87

(51) Int. Cl.$^4$: C 07 D 233/32, C 07 B 57/00

(21) Application number: 83103665.2

(22) Date of filing: 15.04.83

## (54) Method of obtaining optically active half esters.

(30) Priority: 16.04.82 JP 64198/82
11.08.82 JP 140299/82
08.10.82 JP 178103/82
27.04.82 JP 71779/82
17.05.82 JP 83455/82
16.06.82 JP 104585/82

(43) Date of publication of application:
26.10.83 Bulletin 83/43

(45) Publication of the grant of the patent:
08.07.87 Bulletin 87/28

(84) Designated Contracting States:
CH DE FR IT LI

(56) References cited:
EP-A-0 044 158

JOURNAL OF THE CHEMICAL SOCIETY, PERKIN TRANSACTIONS I, 1978 M. CONQUINI et al. "Asymmetric Selection via Addition. Optically Active Allenic Sulphones" pages 247-249

(73) Proprietor: SUMITOMO CHEMICAL COMPANY, LIMITED
15 Kitahama 5-chome Higashi-ku
Osaka-shi Osaka 541 (JP)

(72) Inventor: Ohashi, Naohito
No. 2-406, Ryodo-cho 4-chome
Nishinomiya-shi Hyogo (JP)
Inventor: Shimago, Kozo
No. 10-4-412, Sonehigashi 2-chome
Toyonaka-shi Osaka (JP)
Inventor: Ikeda, Takaharu
No. 10-13-109, Makitsukadai 1-chome
Sakai-shi Osaka (JP)
Inventor: Ishizumi, Kikuo
No. 16-10, Uenonishi 2-chome
Toyonaka-shi Osaka (JP)
Inventor: Katsura, Tadashi
No. 9, Yamadahigashi 2-chome
Suita-shi Osaka (JP)
Inventor: Minai, Masayoshi
No. 1606-5, Harimada-cho
Moriyama-shi Shiga (JP)

(74) Representative: Hansen, Bernd, Dr.rer.nat. et al
Hoffmann, Eitle & Partner Patentanwälte
Arabellastrasse 4
D-8000 München 81 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 092 194 B1

Courier Press, Leamington Spa, England.

(56) References cited:

CHEMICAL REWIENS, vol. 80, no. 3, June 1980 A. COLLET et al. "Optical Resolution by Direct Crystallization of Enantiomer Mixtures" pages 215-230

AGRICULTURAL AND BIOLOGICAL CHEMISTRY, vol. 46 (7), July 1982, S. IRIUCHIJIMA et al. "Asymmetric Hydrolysis of Prochiral Diesters with Pig Liver Esterase. Preparation of Optically Active Intermediates for the Synthesis of (+)-Biotin and (+)-alpha-Methyl-3,4-dihydroxyphenylalanine" pages 1907-1910

## Description

This invention relates to a method for preparing a (4S,5R)-1,3-dibenzyl-5-alkoxy or alkenyloxycarbonyl-2-oxoimidazoline-4-carboxylic acid (Ia) and a (4R,5S)-1,3-dibenzyl-5-alkoxy or alkenyloxycarbonyl-2-oxo-imidazolidine-4-carboxylic acid (Ib), each being represented by the formula (I):

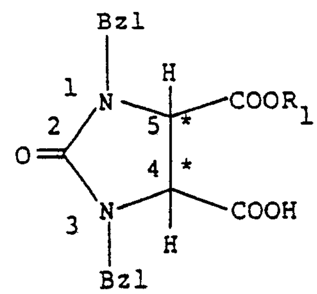

(I)

wherein $R_1$ is an alkyl group or an alkenyl group, each having 1 to 5 carbon atoms; Bzl is a benzyl group; * indicates an asymmetric carbon atom; and the 4- and 5-positions are a cis-configuration.

The optically active half ester obtained by the method of this invention can be selectively reduced in either the lower alkoxycarbonyl group or carboxyl group thereof and cyclized, whereby optically active cis-1,3-dibenzylhexahydro-1H-furo[3,4-d]imidazole-2,4-dione (hereinafter abbreviated as "lactone") represented by the formula (III):

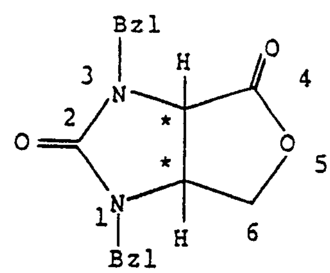

(III)

wherein Bzl is a benzyl group; * indicates an asymmetric carbon atom; and the 3a- and 6a-positions are a cis-configuration, can be produced. The lactone of the formula (III) is known as an important intermediate for the production of biotin.

In the production of the optically active lactone from the optically active half ester, the lactone produced by reducing the lower alkoxy- or alkenyloxycarbonyl group of the optically active half ester and then cyclizing and that produced by reducing the carboxyl group of the optically active half ester and then cyclizing are in reverse in respect to the configuration. In other words, when the optically active half ester having a (4S,5R)-configuration is concerned, if the lower alkoxy- or alkenyloxycarbonyl group thereof is reduced and cyclized, the resulting optically active lactone has a (3aS,6aR)-configuration, whereas if the carboxyl group thereof is reduced and cyclized, the resulting optically active lactone has a (3aR,6aS)-configuration. On the other hand, when the optically active half ester having a (4R,5S)-configuration is concerned, if the lower alkoxy- or alkenyloxycarbonyl group thereof is reduced and cyclized, the resulting optically active lactone hasa (3aR,6aS)-configuration, whereas if the carboxyl group thereof is reduced and cyclized, the resulting optically active lactone has a (3aS,6aR)-configuration.

Such is illustrated in the following scheme.

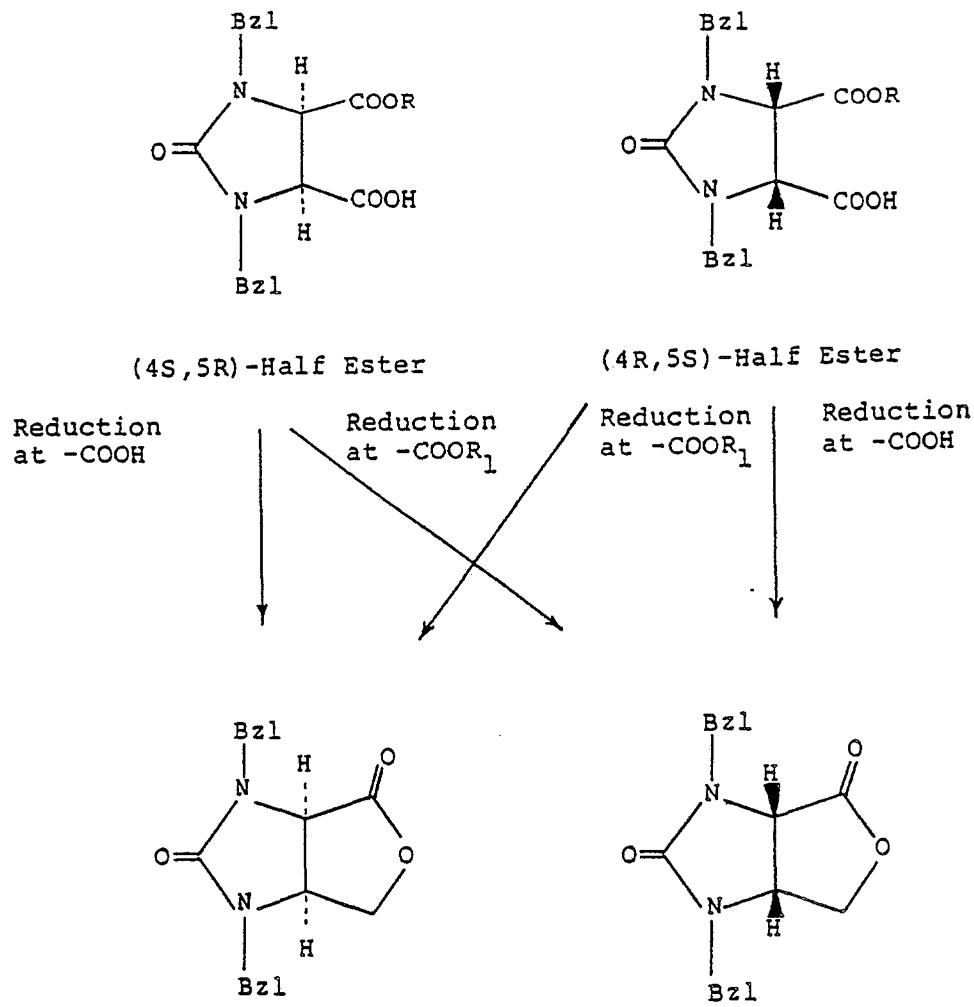


(4S,5R)-Half Ester

(4R,5S)-Half Ester

(3aR,6aS)-Lactone

(3aS,6aR)-Lactone

Accordingly, from any of the optically active (Ia) and (Ib) obtained in this invention, the optically active lactone having a (3aS,6aR)-configuration which is an important intermediate for the production of natural-type biotin can be derived. In short, any of the two optically active half esters obtained in this invention can be used as an intermediate for the production of natural-type biotin.

Such is meaningful and valuable on an industrial scale because in the usual optical resolution operation, only either one of the two optical isomers is utilized and the other is racemized and subjected to further optical resolution. Further, (Ib) is important as a starting material of a resolution agent for the production of an optically active isomer of a 4-hydroxy-2-cyclopentenone which is an intermediate for the production of prostaglandins.

The mixture of the half esters (Ia) and (Ib) can be obtained by, for example, heating a cis-1,3-dibenzyl-2-oxoimidazolidine-4,5-dicarboxylic acid and a corresponding alcohol, or by other methods (Japanese Patent Application (OPI) No. 172257/82). But, there have not yet been known any methods for separating the half esters (Ia) and (Ib) from the mixture thereof.

Of these half esters, only (4S,5R)-1,3-dibenzyl-5-methoxycarbonyl- or 5-n-propoxycarbonyl-2-oxo-imidazolidine-4-carboxylic acid is known by the resume of lecture for the 1982's meeting in The Agricultural Chemical Society of Japan, page 391, in more detail, *Agric. Biol. Chem.*, 46 (7), pp 1907—1910 (1982).

Such optically active compounds can be prepared by enzymatic method from cis-1,3-dibenzyl-4,5-dimethoxycarbonyl- or 4,5-di-n-propoxycarbonyl-2-oxoimidazolidine.

As the result of extensive investigations, the present inventors found a method of optically resolving a mixture of the half esters (Ia) and (Ib) whereby (Ia) and (Ib) can be easily separated from each other, which method is quite different from the above-described known method, and attained this invention.

The invention comprises a method for preparing optically active half esters of the formula

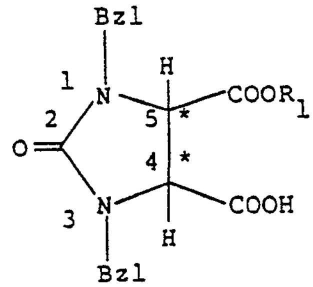

(I)

wherein $R_1$ is an alkyl group or an alkenyl group, each having 1 to 5 carbon atoms; Bzl is a benzyl group; * indicates an asymmetric carbon atom; and the 4- and 5-positions are a cis-configuration, characterized in that a mixture of a (4S,5R)-1,3-dibenzyl-5-alkoxy or alkenyloxycarbonyl-2-oxoimidazolidine-4-carboxylic acid and a (4R,5S)-1,3-dibenzyl-5-alkoxy or alkenyloxycarbonyl-2-oxoimidazolidine-4-carboxylic acid, each being represented by the above formula is optically resolved by using an optically active d- or l-amine represented by the formula (II)

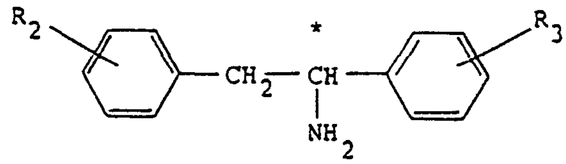

(II)

wherein $R_2$ and $R_3$ are each a hydrogen atom, a halogen atom or a methyl group; and * indicates an asymmetric carbon atom.

$R_1$ in formula (I) is preferably a methyl group, an ethyl group, an n-propyl group or an isopropyl group.

The optically active d- or l-amine (II) is applied to the mixture of the optically active half esters (Ia) and (Ib) to produce crystals by a salt of (Ia) or (Ib) and (II), which are subsequently decomposed to obtain solely the half ester (Ia) or (Ib).

Further, a filtrate from which the deposited crystals have been filtered out is concentrated to obtain a solid. The solid thus obtained is subjected to acid decomposition, and the freed optically active d- or l-amine is separated and removed to obtain a half ester mixture having an excess amount of either the optically active half ester (Ia) or (Ib). The resulting mixture is subjected to preferential crystallization, whereby the desired optically active half ester (Ia) or (Ib) can also be obtained solely.

The method of this invention is described in detail below.

To a mixture of the optically active half esters (Ia) and (Ib) are added predetermined amounts of a solvent and the optically active d- or l-amine (II) to produce a salt of the optically active half esters (Ia) and (Ib) and the optically active d- or l-amine (II). The salt thus produced is dissolved in a solvent.

Illustrative examples of the optically active amine (II) which can be used as an optical resolution agent include optically active isomers of α-phenyl-β-phenylethylamine, α-phenyl-β-(p-tolyl)ethylamine, α-(p-tolyl)-β-phenylethylamine, α-(p-tolyl)-β-(p-tolyl)ethylamine, α-phenyl-β-(p-chlorophenyl)ethylamine, α-(p-chlorophenyl)-β-phenylethylamine, etc., with optically active isomers of α-phenyl-β-phenylethylamine, α-phenyl-β-(p-tolyl)ethylamine and α-(p-tolyl)-β-phenylethylamine being preferred.

The amount of the optically active active amine (II) which can be used is usually about 0.7 to 1.2 mols, preferably 0.8 to 1.1 mols, per mol of the mixture of the optically active half esters (Ia) and (Ib).

In the resolution, a solvent is usually used. Illustrative examples of the solvent which can be used include mixtures of water-soluble organic solvents (e.g., tetrahydrofuran, dioxane, methanol, ethanol, isopropanol, acetone, etc.) and water, and benzene, toluene, chloroform, ethyl acetate, methyl isobutyl ketone, hexane, petroleum ether, etc. and mixtures of these solvents.

The amount of the solvent used is an amount sufficient for fractionally crystallizing the salt of the optically active half esters (Ia) and (Ib) and the optically active d- or l-amine (II) from a dissolved solution thereof.

The reaction temperature can be optionally chosen within the range of from −20°C to the boiling point of the solvent used (but not lower than the melting point of the solvent) and is preferably not lower than the temperature at which the produced salt is crystallized.

By the above reaction, the salt of the optically active half esters (Ia) and (Ib) and the optically active amine (II) is produced and then allowed to gradually cool to thereby crystallize a salt of diastereomer of either optically active isomer.

In this case, in order to promote the crystallization of the salt of diastereomer, it is preferred to add a seed crystal of the same type of a salt of optically active half ester to the mixture system.

The crystal thus produced is separated from the mother liquor and then decomposed with an acid or alkali. When the acid is used, examples of the acid which can be used include hydrochloric acid, sulfuric acid, phosphoric acid, etc. The acid can be used freely if the amount is at least one mol per mol of the

crystal filtered out. But, it is usually within the range of from about 1 to 1.5 mols per mol of the crystal. The decomposition solution is extracted with an organic solvent (e.g., toluene, chloroform, diethyl ether, ethyl acetate, etc., or mixed solvents of these solvents). If desired, the extracted oily layer is washed with water, and the solvent is distilled off. Thus, the one-sided optically active half ester can be obtained.

The aqueous layer obtained by the extraction and the water used for washing the extracted oily layer are neutralized with an alkali (e.g., sodium hydroxide, potassium hydroxide, sodium carbonate, potassium carbonate, etc.) and extracted with an organic solvent (e.g., toluene, chloroform, diethyl ether, ethyl acetate, etc.). The extracted oily layer is washed with water, and the solvent is distilled off. Thus, the optically active d- or l-amine (II) used as the resolution agent can be recovered in a high yield. What the resolution agent can be recovered in a high yield is greatly advantageous in practicing the invention on an industrial scale.

When the alkali is used in the decomposition reaction, examples of the alkali which can be used include sodium hydrogencarbonate, sodium carbonate, potassium carbonate, sodium hydroxide, potassium hydroxide, etc. The decomposition reaction mixture is extracted with an organic solvent, whereby the optically active amine used as the resolution agent is transferred into the organic solvent layer whereas the desired optically active half ester is transferred into the aqueous layer as a salt. The resulting aqueous layer is neutralized with an acid and extracted with an organic solvent. Thus, one-sided optically active half ester can be obtained. In this case, the same acid and organic solvent for extraction as those illustrated above can be used.

A filtrate from which the crystal produced has been removed (if desired, a concentration residue obtained by concentrating the filtrate) is decomposed with the same acid as that described above, and the decomposition solution is extracted with a solvent. The extract is further concentrated to obtain a mixture of the optically active half esters (Ia) and (Ib) having an excess amount of the other-sided optically active half ester. The mixture is crystallized using a suitable solvent, whereby the desired other-sided optically active half ester can be preferentially crystallized. The crystal produced is then isolated. In this case, it is preferred to add a seed crystal of the same type of optically active half ester to the mixture system prior to the crystallization of the other-sided optically active half ester.

Illustrative examples of the solvent which can be used for the preferential crystallization include alcohol solvents (e.g., methanol, ethanol, isopropyl alcohol, etc.), ketone solvents (e.g., acetone, methyl ethyl ketone, etc.), mixed solvents of these solvents and water, aromatic hydrocarbon solvents (e.g., benzene, toluene, etc.), nitrile solvents (e.g., acetonitrile, etc.), and the like. The amount of the solvent used is not particularly restricted, but it is usually 2 to 30 times the weight of the half ester mixture.

Since in the preferential crystallization, the desired optically active half ester can be produced and separated in a yield of higher than the optical excessive rate of optically active half ester, it is quite valuable from the industrial viewpoint.

The thus isolated optically active half esters (Ia) and (Ib) can be purified by recrystallization, etc., if desired.

In the optical resolution of the optically active half esters (Ia) and (Ib), each having a methyl group for $R_1$ in the formula (I), from an optically active isomer of α-phenyl-β-(p-tolyl)ethylamine (hereinafter abbreviated as "PTE") which has a methyl group for $R_2$ and a hydrogen atom for $R_3$, respectively in the formula (II), if a solvent is selected, it is possible to selectively first crystallize either one of the half ester (Ia) or (Ib).

For example, when d-PTE is applied to a mixture of (Ia) and (Ib) in a mixed solvent of a water-soluble organic solvent and water (the mixed solvent being hereinafter referred to as "hydrated solvent"), a salt of (Ib) and d-PTE can be selectively obtained as a crystal. On the other hand, when d-PTE is applied to a mixture of (Ia) and (Ib) in benzene, a salt of (Ia) and d-PTE can be selectively obtained as a crystal. These crystals are respectively subjected to acid decomposition, whereby the respective optically active half esters (Ia) and (Ib) can be isolated.

A filtrate from which the crystal has been filtered out is also recrystallized by altering the solvent, whereby the other-sided optically active half ester can be obtained.

For example, a filtrate obtained by applying d-PTE to a mixture of (Ia) and (Ib) in the hydrated solvent and filtering out the resulting salt of (Ib) and d-PTE is concentrated to obtain a solid which is subsequently recrystallized from benzene to obtain a salt of (Ia) and d-PTE. Then, (Ia) is obtained by acid decomposition of the salt of (Ia) and d-PTE. Or the solid obtained above is subjected to acid decomposition to remove d-PTE, followed by recrystallizing from the same solvent used in the preferential crystallization. Thus, (Ia) can be preferentially obtained.

In contrast, when benzene is used as the solvent in place of the hydrated solvent, if the hydrated solvent is used in the recrystallization of the filtrate, (Ib) can be obtained from the filtrate in a manner quite the same as that above.

When l-PTE is used as the solution agent, the desired optically active half ester can be obtained in the same manner except that the relation of (Ia) and (Ib) is reversed as compared with that when d-PTE is used.

This invention is described in more detail by reference to the following examples, but it is not to be construed that the invention is limited thereto.

Synthesis Example 1

Preparation of Racemic Half Esters:

(1) A mixture of 68.0 g of cis-1,3-dibenzyl-2-oximidazolidine-4,5-dicarboxylic acid anhydride, 800 ml of toluene and 12.4 g of methanol was reacted at 80 to 82°C for 3 hours and then cooled to room temperature. Crystals produced were filtered and dried under reduced pressure to obtain 61.3 g of racemic cis-1,3-dibenzyl-5-methoxycarbonyl-2-oxoimidazolidine-4-carboxylic acid. M.P., 130—131°C.

The above half ester was recrystallized from toluene to give a purified product. M.P., 131—132°C.

(2) A mixture of 70.0 g of cis-1,3-dibenzyl-2-oxoimidazolidine-4,5-dicarboxylic acid anhydride, 16.6 g of 99.5% ethanol and 1000 ml of benzene was heated under reflux for 2 hours. The reaction mixture was concentrated under atmospheric pressure. The residue was stirred overnight with 200 ml of n-hexane. Crystals produced were filtered and dried under reduced pressure to obtain 76.2 g of racemic cis-1,3-dibenzyl-5-ethoxycarbonyl-2-oxoimidazolidine-4-carboxylic acid which were then recrystallized for purification from toluene. M.P., 110—111°C.

Example 1

In a flask were charged 382.4 g (total 1 mol) of a 1:1 mixture of (4S,5R)-1,3-dibenzyl-4-carboxy-5-ethoxycarbonylimidazolidine-2-one and (4R,5S)-1,3-dibenzyl-4-carboxy-5-ethoxycarbonylimidazolidine-2-one, 3l of methanol and 2.5l of water and heated at 50°C. 211.3 g (1 mol) of d-α-phenyl-β-(p-tolyl)ethylamine was added dropwise thereto. The mixture was gradually cooled, and small amounts of crystals of a salt of (4S,5R)-1,3-dibenzyl-4-carboxy-5-ethoxycarbonylimidazolidine-2-one and d-α-phenyl-β-(p-tolyl)ethylamine prepared separately were added thereto as seed crystals at 30 to 31°C. The resulting mixture was further gradually cooled to 20°C and maintained at 20°C for 2 hours. The deposited crystals were filtered and dried. To the resulting crystals were added 500 cc of water and 47.0 g of concentrated hydrochloric acid, and the mixture was subjected to acid decomposition and extraction with 300 cc of ethyl acetate to separate (4S,5R)-1,3-dibenzyl-4-carboxy-5-ethoxycarbonylimidazolidine-2-one. The extract was washed twice with 100 cc of water, and the ethyl acetate was distilled off to obtain 157.5 g (Yield: 41.2%) of (4S,5R)-1,3-dibenzyl-4-carboxy-5-ethoxycarbonylimidazolidine-2-one.

$[\alpha]^{20}_{365} = -20.9°$ (c=1, DMF). M.P., 134—136°C. Optical purity, 96.4%.

Example 2

In a flask were charged 368.4 g (total 1 mol) of a 1:1 mixture of (4S,5R)-1,3-dibenzyl-4-carboxy-5-methoxycarbonylimidazolidine-2-one and (4R,5S)-1,3-dibenzyl-4-carboxy-5-methoxycarbonylimidazolidine-2-one, 3l of acetone and 3l of water and heated at 60°C. 211.3 g (1 mol) of 1-α-phenyl-β-(p-tolyl)ethylamine was added dropwise thereto. The mixture was gradually cooled, and small amounts of crystals of a salt of (4S,5R)-1,3-dibenzyl-4-carboxy-5-methoxycarbonylimidazolidine-2-one and 1-α-phenyl-β-(p-tolyl)ethylamine prepared separately were added thereto as seed crystals at 34 to 35°C. The resulting mixture was further gradually cooled to 15°C and maintained at 15°C for 2 hours. The deposited crystals were filtered and dried. To the resulting crystals were added 500 cc of water and 46 g of concentrated hydrochloric acid, and the mixture was subjected to acid decomposition and extraction with 300 cc of ethyl acetate to separate (4S,5R)-1,3-dibenzyl-4-carboxy-5-methoxycarbonylimidazolidine-2-one. The extract was washed twice with 100 cc of water, and the ethyl acetate was distilled off to obtain 154.4 g (Yield: 41.9%) of (4S,5R)-1,3-dibenzyl-4-carboxy-5-methoxycarbonylimidazolidine-2-one.

$[\alpha]^{25}_{365} = -28.3°$ (c=1, DMF). M.P., 148—149°C. Optical purity, 99%.

The filtrate was concentrated to obtain a solid. To the thus obtained solid were added 600 cc of water and 64 g of concentrated hydrochloric acid, followed by subjecting to acid decomposition. 300 cc of ethyl acetate was then added thereto to extract and separate a mixture of (4S,5R)-1,3-dibenzyl-4-carboxy-5-methoxycarbonylimidazolidine-2-one and (4R,5S)-1,3-dibenzyl-4-carboxy-5-methoxycarbonylimidazolidine-2-one containing an excess amount of (4R,5S)-1,3-dibenzyl-4-carboxy-5-methoxycarbonylimidazolidine-2-one. The extract was washed twice with water, and the ethyl acetate was distilled off. A solid obtained was dissolved in 6l of benzene. Thereafter, the solution was gradually cooled and seeded with as a seed crystal (4R,5S)-1,3-dibenzyl-4-carboxy-5-methoxycarbonylimidazolidine-2-one to thereby preferentially crystallize (4R,5S)-1,3-dibenzyl-4-carboxy-5-methoxycarbonylimidazolidine-2-one. After gradually cooling to 35°C, the system was maintained at 35°C for 2 hours. The deposited crystals were filtered and dried to obtain 175.0 g (Yield: 47.5%) of (4R,5S)-1,3-dibenzyl-4-carboxy-5-methoxycarbonylimidazolidine-2-one.

$[\alpha]^{25}_{365} = +27.4°$ (c=1, DMF). M.P., 148—149°C. Optical purity, 97.5%.

Example 3

In a flask were charged 396.4 g (total 1 mol) of a 1:1 mixture of (4S,5R)-1,3-dibenzyl-4-carboxy-5-isopropoxycarbonylimidazolidine-2-one and (4R,5S)-1,3-dibenzyl-4-carboxy-5-isopropoxycarbonylimidazolidine-2-one, 2.5l of isopropanol and 2.5l of water and heated at 50°C. 211.3 g (1 mol) of d-α-phenyl-β-(p-tolyl)ethylamine was added dropwise thereto. The mixture was gradually cooled, and small amounts of crystals of a salt of (4S,5R)-1,3-dibenzyl-4-carboxy-5-isopropoxycarbonylimidazolidine-2-one and d-α-phenyl-β-(p-tolyl)ethylamine prepared were added thereto as seed crystals at 31 to 32°C. The resulting mixture was further gradually cooled to 10°C and maintained at 10°C for 2 hours. The deposited crystals

were filtered and dried. To the resulting crystals were added 500 cc of water and 50 g of concentrated hydrochloric acid, and the mixture was subjected to acid decomposition and extraction with 300 cc of ethyl acetate to separate (4S,5R)-1,3-dibenzyl-4-carboxy-5-isopropoxycarbonylimidazolidine-2-one. The extract was washed twice with 100 cc of water, and the ethyl acetate was distilled off to obtain 179.2 g (Yield: 45.2%) of (4S,5R)-1,3-dibenzyl-4-carboxy-5-isopropoxycarbonylimidzolidine-2-.

$[\alpha]_{365}^{20} = -26.9°$ (c=1, DMF). M.P., 140—142°C. Optical purity, 98.7%.

Example 4

In a flask were charged 368.4 g (total 1 mol) of a 1:1 mixture of (4S,5R)-1,3-dibenzyl-4-carboxy-5-methoxycarbonylimidazolidine-2-one and (4R,5S)-1,3-dibenzyl-4-carboxy-5-methoxycarbonylimidazolidine-2-one, 1,5l of isopropanol and 1.5l of water and heated at 50°C. 197.3 g (1 mol) of d-α-phenyl-β-phenylethylamine was added dropwise thereto. The mixture was gradually cooled, and small amounts of crystals of a salt of (4R,5S)-1,3-dibenzyl-4-carboxy-5-methoxycarbonylimidazolidine-2-one and d-α-phenyl-β-phenylethylamine prepared separately were added thereto as seed crystals at 35°C. The resulting mixture was further gradually cooled to 20°C and maintained at 20°C for 2 hours. The deposited crystals were filtered and dried. To the resulting crystals were added 500 cc of water and 46.5 g of concentrated hydrochloric acid, and the mixture was subjected to acid decomposition and extraction with 300 cc of ethyl acetate to separate (4R,5S)-1,3-dibenzyl-4-carboxy-5-methoxycarbonylimidazolidine-2-one. The extract was washed twice with 100 cc of water, and the ethyl acetate was distilled off to obtain 155.1 g (Yield: 42.1%) of (4R,5S)-1,3-dibenzyl-4-carboxy-5-methoxycarbonylimidazolidine-2-one.

$[\alpha]_{365}^{20} = +27.8°$ (c=1, DMF). M.P., 148—149°C. Optical purity, 98.9%.

Example 5

In a flask were charged 368.4 g (total 1 mol) of a 1:1 mixture of (4S,5R)-1,3-dibenzyl-4-carboxy-5-methoxycarbonylimidazolidine-2-one and (4R,5S)-1,3-dibenzyl-4-carboxy-5-methoxycarbonylimidazolidine-2-one, 2l of methanol and 2l of water and heated at 60°C. 211.3 g (1 mol) of d-α-phenyl-β-(p-tolyl)ethylamine was added dropwise thereto. The mixture was gradually cooled, and small amounts of crystals of a salt of (4R,5S)-1,3-dibenzyl-4-carboxy-5-methoxycarbonylimidazolidine-2-one and d-α-phenyl-β-(p-tolyl)ethylamine prepared separately were added thereto as seed crystals at 40°C. The resulting mixture was further gradually cooled to 25°C and maintained at 25°C for 2 hours. The deposited crystals were filtered and dried. To the resulting crystals were added 500 cc of water and 47.0 g of concentrated hydrochloric acid, and the mixture was subjected to acid decomposition and extraction with 300 cc of ethyl acetate. The extract was washed twice with 100 cc of water, and the ethyl acetate was distilled off to obtain 159.1 g (Yield: 43.2%) of (4R,5S)-1,3-dibenzyl-4-carboxy-5-methoxycarbonylimidazolidine-2-one.

$[\alpha]_{365}^{20} = +27.7°$ (c=1, DMF). M.P., 148—149°C. Optical purity, 98.7%.

The filtrate was concentrated to obtain a solid which was subsequently dissolved in 1.7l of benzene. The solution was gradually cooled to 20°C, and small amounts of crystals of a salt of (4S,5R)-1,3-dibenzyl-4-carboxy-5-methoxycarbonylimidazolidine-2-one and d-α-phenyl-β-(p-tolyl)ethylamine prepared separately were added thereto as seed crystals. The mixture was maintained at 8°C for 2 hours. The deposited crystals were filtered and dried. To the resulting crystals were added 500 cc of water and 47 g of concentrated hydrochloric acid, and the mixture was subjected to acid decomposition and extraction with 300 cc of ethyl acetate. The organic layer was washed twice with 100 cc of water, and the ethyl acetate was distilled off to obtain 157.1 g (Yield: 42.7%) of (4S,5R)-1,3-dibenzyl-4-carboxy-5-methoxycarbonylimidazolidine-2-one.

$[\alpha]_{365}^{20} = -28.4°$ (c=l, DMF). M.P., 148.5—149.5°C. Optical purity, 99.6%.

Example 6

In a flask were charged 368.4 g (total 1 mol) of a 1:1 mixture of (4S,5R)-1,3-dibenzyl-4-carboxy-5-methoxycarbonylimidazolidine-2-yl and (4R,5S)-1,3-dibenzyl-4-carboxy-5-methoxycarbonylimidazolidine-2-one, 3l of acetone and 3l of water and heated at 60°C. 211.3 g (1 mol) of 1-α-phenyl-β-(p-tolyl)ethylamine was added dropwise thereto. The mixture was gradually cooled, and small amounts of crystals of a salt of (4S,5R)-1,3-dibenzyl-4-carboxy-5-methoxycarbonylimidazolidine-2-one and 1-α-phenyl-β-(p-tolyl)-ethylamine prepared separately were added thereto as seed crystals at 34 to 35°C. The resulting mixture was further gradually cooled to 15°C and maintained at 15°C for 2 hours. The deposited crystals were filtered and dried. To the resulting crystals were added 500 cc of water and 46 g of concentrated hydrochloric acid, and the mixture was subjected to acid decomposition and extraction with 300 cc of ethyl acetate. The organic layer was washed twice with 100 cc of water, and the ethyl acetate was distilled off to obtain 154.4 g (Yield: 41.9%) of (4S,5R)-1,3-dibenzyl-4-carboxy-5-methoxycarbonylimidazolidine-2-one.

$[\alpha]_{365}^{20} = -28.3°$ (c=1, DMF). M.P. 148—149°C. Optical purity, 99%.

The filtrate was concentrated to obtain a solid. To the thus obtained solid were added 600 cc of water and 64 g of concentrated hydrochloric acid, followed by subjecting to acid decomposition. 300 cc of ethyl acetate was then added thereto to extract and separate a mixture of (4S,5R)-1,3-dibenzyl-4-carboxy-5-methoxycarbonylimidazolidine-2-one and (4R,5S)-1,3-dibenzyl-4-carboxy-5-methoxycarbonylimidazolidine-2-one containing an excess amount of (4R,5S)-1,3-dibenzyl-4-carboxy-5-methoxycarbonylimidazolidine-2-one. The extract was washed twice with water, and the ethyl acetate was distilled off. A solid obtained was dissolved in 6 of benzene. Thereafter, the solution was gradually cooled and seeded

with as a seed crystal (4R,5S)-1,3-dibenzyl-4-carboxy-5-methoxycarbonylimidazolidine-2-one to thereby preferentially crystallize (4R,5S)-1,3-dibenzyl-4-carboxy-5-methoxycarbonylimidazolidine-2-one. After gradually cooling to 15°C, the system was maintained at 15°C for 2 hours. The deposited crystals were filtered and dried to obtain 175.0 g (Yield: 47.5%) of (4R,5S)-1,3-dibenzyl-4-carboxy-5-methoxycarbonylimidazolidine-2-one.

$[\alpha]^{20}_{365}$ = +27.4° (c=1, DMF). M.P., 148—149°C. Optical purity, 97.6%.

Further, the filtrate was concentrated to obtain a solid which was subsequently dissolved in 1l of benzene. Thereafter, the solution was gradually cooled and seeded with as a seed crystal (4S,5R)-1,3-dibenzyl-4-carboxy-5-methoxycarbonylimidazolidine-2-one to thereby preferentially crystallize (4S,5R)-1,3-dibenzyl-4-carboxy-5-methoxycarbonylimidazolidine-2-one. After gradually cooling to 15°C, the system was maintained at 15°C for 2 hours. Crystals produced were filtered and dried to obtain 27.6 g (Yield: 7.5%) of (4S,5R)-1,3-dibenzyl-4-carboxy-5-methoxycarbonylimidazolidine-2-one.

$[\alpha]^{20}_{365}$ = −28.0° (c=1, DMF). M.P., 148—149°C. Optical purity, 99.1%.

## Example 7

In a flask were charged 368.4 g (total 1 mol) of a 1:1 mixture of (4S,5R)-1,3-dibenzyl-4-carboxy-5-methoxycarbonylimidazolidine-2-one and (4R,5S)-1,3-dibenzyl-4-carboxy-5-methoxycarbonylimidazolidine-2-one and 3l of benzene and heated at 30°C. 211.3 g (1 mol) of d-α-phenyl-β-(p-tolyl)ethylamine was added dropwise thereto. The mixture was gradually cooled, and small amounts of crystals of a salt of (4S,5R)-1,3-dibenzyl-4-carboxy-5-methoxycarbonylimidazolidine-2-one and d-α-phenyl-β-(p-tolyl)ethylamine prepared separately were added thereto as seed crystals at 20°C. The resulting mixture was further gradually cooled to 5°C and maintained at 5°C for 2 hours. The deposited crystals were filtered and dried. To the resulting crystals were added 500 cc of water and 50 g of concentrated hydrochloric acid, and the mixture was subjected to acid decomposition and extraction with 300 cc of ethyl acetate. The extract was washed twice with 100 cc of water, and the ethyl acetate was distilled off to obtain 167.3 g (Yield: 45.4%) of (4S,5R)-1,3-dibenzyl-4-carboxy-5-methoxycarbonylimidazolidine-2-one.

$[\alpha]^{20}_{365}$ = −28.0° (c=1, DMF). M.P., 149—150°C. Optical purity, 99%.

The filtrate was concentrated to obtain a solid. To the solid was added a mixed solvent of 1.35l of ethanol and 0.67l of water, and the mixture was heated at 50°C for dissolution. The resulting solution was cooled to 10°C and maintained at 10°C for 2 hours. The deposited crystals were filtered and dried, followed by subjecting to acid decomposition and extraction with 300 cc of ethyl acetate. The extract was washed twice with 100 cc of water, and the ethyl acetate was distilled off to obtain 156.6 g (Yield: 42.5%) of (4R,5S)-1,3-dibenzyl-4-carboxy-5-methoxycarbonylimidazolidine-2-one.

$[\alpha]^{20}_{365}$ = +27.6° (c=1, DMF). M.P., 148—149°C. Optical purity, 98.6%.

## Example 8

In a flask were charged 382.4 g (total 1 mol) of a 1:1 mixture of (4S,5R)-1,3-dibenzyl-4-carboxy-5-ethoxycarbonylimidazolidine-2-one and (4R,5S)-1,3-dibenzyl-4-carboxy-5-ethoxycarbonylimidazolidine-2-one, 2.5l of isopropanol and 2.5l of water and heated at 50°C. 211.3 g (1 mol) of d-α-phenyl-β-(p-tolyl)ethylamine was added dropwise thereto. The mixture was gradually cooled, and small amounts of crystals of a salt of (4S,5R)-1,3-dibenzyl-4-carboxy-5-ethoxycarbonylimidazolidine-2-one and d-α-phenyl-β-(p-tolyl)ethylamine prepared separately were added thereto as seed crystals at 30 to 31°C. The resulting mixture was further gradually cooled to 10°C and maintained at 10°C for 2 hours. The deposited crystals were filtered out.

The filtrate was concentrated, and 600 cc of water and 62 g of concentrated hydrochloric acid were added thereto, followed by subjecting to acid decomposition. 400 cc of ethyl acetate was then added thereto to extract and separate a mixture of (4R,5S)-1,3-dibenzyl-4-carboxy-5-ethoxycarbonylimidazolidine-2-one and (4S,5R)-1,3-dibenzyl-4-carboxy-5-ethoxycarbonylimidazolidine-2-one containing an excess amount of (4R,5S)-1,3-dibenzyl-4-carboxy-5-ethoxycarbonylimidazolidine-2-one. The extract was washed twice with 100 cc of water, and the ethyl acetate as distilled off. A solid obtained was dissolved in 1l of toluene. Thereafter, the seed solution was gradually cooled and seeded with as a seed crystal (4R,5S)-1,3-dibenzyl-4-carboxy-5-ethoxycarbonylimidazolidine-2-one to thereby preferentially crystallize (4R,5S)-1,3-dibenzyl-4-carboxy-5-ethoxycarbonylimidazolidine-2-one. After gradually cooling to 20°C, the system was maintained at 20°C for 2 hours. The deposited crystals were filtered and dried to obtain 171.3 g (Yield: 44.8%) of (4R,5S)-1,3-dibenzyl-4-carboxy-5-ethoxycarbonylimidazolidine-2-one.

$[\alpha]^{20}_{365}$ = +21.1° (c=1, DMF). M.P., 135—136°C. Optical purity, 97.6%.

## Example 9

In a flask were charged 396.4 g (total 1 mol) of a 1:1 mixture of (4S,5R)-1,3-dibenzyl-4-carboxy-5-isopropoxycarbonylimidazolidine-2-one and (4R,5S)-1,3-dibenzyl-4-carboxy-5-isopropoxycarbonylimidazolidine-2-one, 2.5l of methanol and 2.5l of water and heated at 50°C. 211.3 g (1 mol) of d-α-phenyl-β-(p-tolyl)ethylamine was added dropwise thereto. The mixture was gradually cooled, and small amounts of crystals of a salt of (4S,5R)-1,3-dibenzyl-4-carboxy-5-isopropoxycarbonylimidzolidine-2-one and d-α-phenyl-β-(p-tolyl)ethylamine prepared separately were added thereto as seed crystals at 31 to 32°C. The

resulting mixture was further gradually cooled to 10°C and maintained at 10°C for 2 hours. The deposited crystals were filtered out.

The filtrate was concentrated, and 600 cc of water and 63 g of concentrated hydrochloric acid were added thereto, followed by subjecting to acid decomposition. 400 cc of ethyl acetate was then added thereto to extract and separate a mixture of (4R,5S)-1,3-dibenzyl-4-carboxy-5-isopropoxycarbonylimidazolidine-2-one and (4S,5R)-1,3-dibenzyl-4-carboxy-5-isopropoxycarbonylimidazolidine-2-one containing an excess amount of (4R,5S)-1,3-dibenzyl-4-carboxy-5-isopropoxycarbonylimidazolidine-2-one. The extract was washed twice with 100 cc of water, and the ethyl acetate was distilled off. A solid obtained was dissolved in 1l of toluene. Thereafter, the solution was gradually cooled and seeded with as a seed crystal (4R,5S)-1,3-dibenzyl-4-carboxy-5-isopropoxycarbonylimidazolidine-2-one to thereby preferentially crystallize (4R,5S)-1,3-dibenzyl-4-carboxy-5-isopropoxycarbonylimidazolidine-2-one. After gradually cooling to 10°C, the system was maintained at 10°C for 2 hours. The deposited crystals were filtered and dried to obtain 167.7 g (Yield: 42.3%) of (4R,5S)-1,3-dibenzyl-4-carboxy-5-isopropoxyimidazolidine-2-one.

$[\alpha]^{20}_{365}$ = +26.7° (c=1, DMF). M.P., 140—142°C. Optical purity, 98.5%.

Example 10

In a flask were charged 396.4 g (total 1 mol) of 1:1 mixture of (4S,5R)-1,3-dibenzyl-4-carboxy-5-isopropoxycarbonylimidazolidine-2-one and (4R,5S)-1,3-dibenzyl-4-carboxy-5-isopropoxycarbonylimidazolidine-2-one, 2.5l of isopropanol and 2.5l of water and heated at 50°C. 211.3 g (1 mol) of 1-α-phenyl-β-(p-tolyl)ethylamine was added dropwise thereto. The mixture was gradually cooled, and small amounts of crystals of a salt of (4R,5S)-1,3-dibenzyl-4-carboxy-5-isopropoxycarbonylimidazolidine-2-one and 1-α-phenyl-β-(p-tolyl)ethylamine prepared separately were added thereto as seed crystals at 30 to 31°C. The resulting mixture was further gradually cooled to 10°C and maintained at 10°C for 2 hours. The deposited crystals were filtered and dried. To the resulting crystals were added 500 cc of water and 50 g of concentrated hydrochloric acid, and the mixture was subjected to acid decomposition and extraction with 400 cc of ethyl acetate to separate (4R,5S)-1,3-dibenzyl-4-carboxy-5-isopropoxycarbonylimidazolidine-2-one. The extract was washed twice with 100 cc of water, and the ethyl acetate was distilled off to obtain 169.7 g (Yield: 42.8%) of (4R,5S)-1,3-dibenzyl-4-carboxy-5-isopropoxycarbonylimidazolidine-2-one.

$[\alpha]^{20}_{365}$ = +26.6° (c=1, DMF). M.P., 142—144°C. Optical purity, 98.4%.

Example 11

In a flask were charged 396.4 g (total 1 mol) of a 1:1 mixture of (4S,5R)-1,3-dibenzyl-4-carboxy-5-n-propoxycarbonylimidazolidine-2-one and (4R,5S)-1,3-dibenzyl-4-carboxy-5-n-propoxycarbonylimidazolidine-2-one, 2.5l of isopropanopl and 2.5l of water and heated at 60°C. 211.3 g (1 mol) of 1-α-phenyl-β-(p-tolyl)ethylamine was added dropwise thereto. The mixture was gradually cooled, and small amounts of crystals of a salt of (4R,5S)-1,3-dibenzyl-4-carboxy-5-n-propoxycarbonylimidazolidine-2-one and 1-α-phenyl-β-(p-tolyl)ethylamine prepared separately were added thereto as seed crystals at 30°C. The resulting mixture was further gradually cooled to 10°C and maintained at 10°C for 2 hours. The deposited crystals were filtered and dried. To the resulting crystals were added 500 cc of water and 50 g of concentrated hydrochloric acid, and the mixture was subjected to acid decomposition. Thereafter, 400 cc of ethyl acetate was added thereto to obtain 155.0 g (Yield: 39.1%) of (4R,5S)-1,3-dibenzyl-4-carboxy-5-n-propoxycarbonylimidazolidine-2-one.

$[\alpha]^{20}_{365}$ = +19.2° (c=1, DMF). M.P., 126—127°C. Optical purity, 96.1%.

The optical purity in each of the Examples was measured in such a manner that the optically active half ester was converted to an acid chloride by using oxalyl chloride and reacted with d-phenylethylamine to introduce into a diastereomer of an amide of d-phenylethylamine followed by separating by liquid chromatography and measuring.

**Claims**

1. A method for preparing optically active half esters of the formula

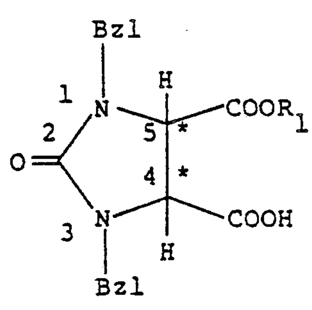

(I)

wherein $R_1$ is an alkyl group or an alkenyl group, each having 1 to 5 carbon atoms; Bzl is a benzyl group; * indicates an asymmetric carbon atom; and the 4- and 5-positions are a cis-configuration, characterized in

that a mixture of a (4S,5R)-1,3-dibenzyl-5-alkoxy or alkenyloxycarbonyl-2-oxoimidazolidine-4-carboxylic acid and a (4R,5S)-1,3-dibenzyl-5-alkoxy or alkenyloxycarbonyl-2-oxoimidazolidine-4-carboxylic acid, each being represented by the above formula is optically resolved by using an optically active d- or l-amine represented by the formula (II)

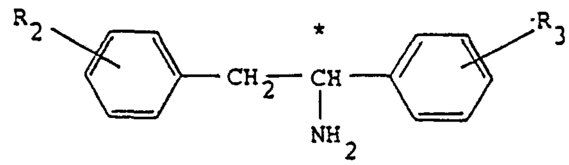

(II)

wherein $R_2$ and $R_3$ are each a hydrogen atom, a halogen atom or a methyl group; and * indicates an asymmetric carbon atom.

2. A method according to Claim 1, wherein $R_1$ is a methyl group, an ethyl group, an n-propyl group or an isopropyl group.

3. A method according to Claim 1, wherein said optically active d- or l-amine is an optically active isomer of α-phenyl-β-(p-tolyl)ethylamine, α-(p-tolyl)-β-phenylethylamine or α-phenyl-β-phenylethylamine.

4. A method according to Claim 1, wherein the amount of said optically active d- or l-amine is about 0.7 to 1.2 mols per mol of the starting half ester.

**Patentansprüche**

1. Verfahren zur Herstellung von optisch aktiven Halbestern der Formel

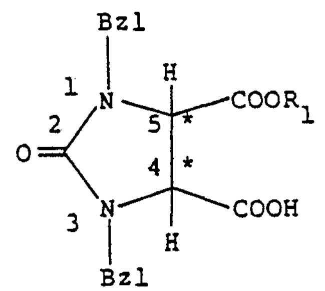

(I)

worin $R_1$ eine Alkylgruppe oder eine Alkenylgruppe mit jeweils 1 bis 5 Kohlenstoffatomen bedeutet; Bzl eine Benzylgruppe ist; * ein asymmetrisches Kohlenstoffatom anzeigt; und die 4- und 5-Stellungen in cis-Konfiguration vorliegen; dadurch gekennzeichnet, dass eine Mischung aus (4S,5R)-1,3-Dibenzyl-5-alkoxy- oder Alkenyloxycarbonyl-2-oxoimidazolidin-4-carboxylsäure und eine (4R,5S)-1,3-Dibenzyl-5-alkoxy- oder Alkenloxycarbonyl-2-oxoimidazolidin-4-carboxylsäure, wie sie jeweils durch die obige Formel dargestellt sind, optisch unter Verwendung eines optisch aktiven d- oder l-Amins der Formel (II)

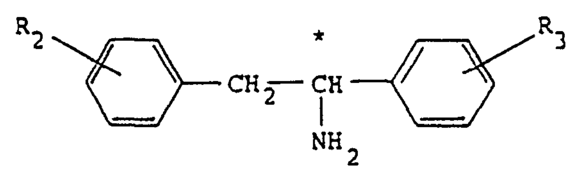

(II)

worin $R_2$ und $R_3$ jeweils ein Wasserstoffatom, ein Halogenatom oder eine Methylgruppe bedeuten und * ein asymmetrisches Kohlenstoffatom anzeigt, gespalten wird.

2. Verfahren gemäss Anspruch 1, bei dem $R_1$ eine Methylgruppe, eine Ethylgruppe, eine n-Propylgruppe oder eine Isopropylgruppe bedeutet.

3. Verfahren gemäss Anspruch 1, bei dem das optisch aktive d- oder l-Amin ein optisch aktives Isomer von alpha-Phenyl-β-(p-tolyl)ethylamin, alpha-(p-Tolyl)-β-phenylethylamin oder alpha-Phenyl-β-phenylethylamin ist.

4. Verfahren gemäss Anspruch 1, bei dem die Menge des optisch aktiven d- oder l-Amins etwa 0,7 bis 1,2 Mol pro Mol des Ausgangs-Halbesters beträgt.

**Revendications**

1. Un procédé pour fabriquer des semi-esters optiquement actifs de formule:

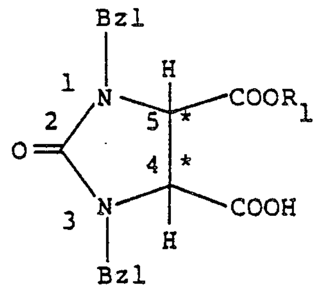

(I)

dans laquelle $R_1$ est un groupe alkyle ou un groupe alcényle, chacun en $C_1$—$C_5$; Bzl est un groupe benzyle; l'astérisque indique un atome de carbone asymétrique; et les positions 4 et 5 sont dans la configuration cis, caractérisé en ce que l'on effectue la résolution optique d'un mélange d'un acide (4S,5R)-1,3-dibenzyl-5-(alcoxy ou alcényloxy)-carbonyl-2-oxoimidazolidine-4-carboxylique et d'un acide (4R,5S)-1,3-dibenzyl-5-(alcoxy ou alcényloxy)-carbonyl-2-oxoimidazolidine-4-carboxylique, représentés chacun par la formule ci-dessus, en utilisant une d- ou l-amine optiquement active représentée par la formule (II)

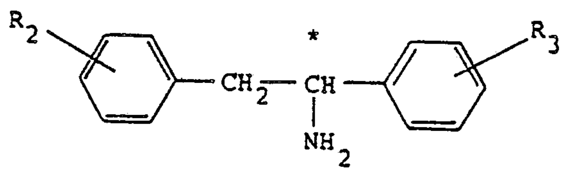

(II)

dans laquelle $R_2$ et $R_3$ représentent chacun un atome d'hydrogène, un atome d'halogène ou un groupe méthyle; et l'astérisque indique un atome de carbone asymétrique.

2. Un procédé selon la revendication 1, dans lequel $R_1$ est un groupe méthyle, éthyle, n-propyle ou isopropyle.

3. Un procédé selon la revendication 1, dans lequel ladite d- ou l-amine optiquement active est un isomère optiquement actif de l'α-phényl-β-(p-tolyl)-éthylamine, de l'α-(p-tolyl)-β-phényléthylamine ou de l'α-phényl-β-phényléthylamine.

4. Un procédé selon la revendication 1, dans lequel la quantité de ladite d- ou l-amine optiquement active est d'environ 0,7 à 1,2 mole par mole du semi-ester de départ.